Europäisches Patentamt

European Patent Office

Office européen des brevets

(11) Numéro de publication: **0 356 304**
**A2**

## (12) DEMANDE DE BREVET EUROPEEN

(21) Numéro de dépôt: 89402255.7

(22) Date de dépôt: 09.08.89

(51) Int. Cl.⁵: **C 07 H 13/08**
C 07 H 13/00, C 07 H 13/04,
C 07 H 5/02

(30) Priorité: 10.08.88 FR 8810785

(43) Date de publication de la demande:
28.02.90 Bulletin 90/09

(84) Etats contractants désignés:
BE CH DE ES FR GB IT LI NL

(71) Demandeur: BEGHIN-SAY SOCIETE ANONYME
F-59239 Thumeries (FR)

(72) Inventeur: Mentech, Julio
1d, rue Phelypeaux
F-69100 Villeurbanne (FR)

(74) Mandataire: Dorland, Anne-Marie et al
BEGHIN-SAY 54, avenue Hache
F-75008 Paris (FR)

(54) Procédé pour la préparation de 4,1',6'-trichloro-4,1',6'-tridésoxy-galactosaccharose.

(57) Procédé de préparation du 4,1',6'-trichloro-4,1',6'-tridéso-xygalactosaccharose (TGS) par protection du saccharose en position 6, chloration dans les positions 4, 1' et 6' avec inversion de configuration au niveau du carbone 4 et déprotection de la position 6 pour donner le TGS visé, dans lequel ladite protection du saccharose en position 6 est effectuée au moyen d'une réaction d'acylation du saccharose, caractérisé par le fait que ladite réaction d'acylation est effectuée avec un système réactif composé d'une phosphine, d'un ester de type azodicarboxylique et d'un composé de type acide.

Le procédé de l'invention permet de préparer le TGS, qui est un édulcorant puissant, sans avoir recours à la séparation coûteuse du 6-monoacylate des autres acylates formés du stade de l'acylation effectuée dans les procédés de l'art antérieur.

EP 0 356 304 A2

**Description**

## PROCEDE POUR LA PREPARATION DE 4,1′,6′-TRICHLORO-4,1′,6′-TRIDESOXY-GALACTOSACCHAROSE

La présente invention concerne un procédé de préparation du 1,6-dichloro-1,6-didésoxy-β- $\underline{D}$ -fructofuranosyl-4-chloro-4-désoxy-α- $\underline{D}$ -galactopyranosyle, appelé couramment 4,1′,6′-trichloro-4,1′, 6′-tridésoxy-galactosaccharose.

Le 4,1′,6′-trichloro-4,1′6′-tridésoxy-galactosaccharose, également connu sous la dénomination de TGS (qui sera souvent utilisée dans la description ci-après) ou encore de sucralose, est un édulcorant puissant, dont le pouvoir sucrant est plusieurs centaines de fois celui du saccharose. Son utilisation comme édulcorant ainsi que des compositions le contenant sont décrites dans le GB-A-1543167.

La préparation de TGS met en jeu la chloration avec inversion de configuration d'un des cinq hydroxyles secondaires de la molécule de saccharose, l'hydroxyle en position 4, ainsi que la chloration de deux des trois hydroxyles primaires, les positions 1′ et 6′. Cette sélection particulière a pu être obtenue par FAIRCLOUGH et al (Carbohydr. Research 1975, 40, 285-298) ainsi que dans le GB-A-1543167 et l'EP-A-0031651, après tritylation du saccharose conduisant au dérivé 6,1′,6′-tritylé. Après acétylation des OH résiduels, détritylation et migration du groupement 4-acétyle vers la position 6, on obtient le 2,3,6,3′,4′-penta-O-acétylsaccharose qui est ensuite chloré par un réactif du type Vilsmeier ou avec du chlorure de sulfuryle. Après désacétylation, on obtient le dérivé visé, c'est-à-dire le TGS.

Une amélioration récente de ce procédé, concernant surtout l'étape de migration d'acétyle a été proposée dans l'EP-A-022907. La séquence réactionnelle mettant en jeu la détritylation et la migration simultanée de l'acétyle comporte un nombre d'étapes relativement élevé. En outre, la réaction de tritylation initiale rend un tel type de procédé onéreux et de ce fait peu intéressant industriellement.

Des recherches se sont donc dirigées vers une autre voie d'accès au TGS qui consisterait à protéger la position primaire 6, à chlorer le dérivé monoprotégé avec un réactif capable d'introduire un atome de chlore à la place des hydroxyles dans les positions 1′, 4 et 6′, avec inversion de configuration au niveau de l'atome de carbone en position 4, et enfin à déprotéger l'hydroxyle en position 6 pour obtenir le TGS.

A cet égard, plusieurs procédés de fabrication du TGS utilisant une telle protection de la position 6 du saccharose ont été proposés mais dans aucun d'eux cette protection n'est régiosélective. Ainsi, le procédé revendiqué dans l'US-A-4380476 (correspondant à l'EP-A-0043649) comprend la mise en réaction du saccharose avec un agent d'acylation dans des conditions telles que l'on obtienne un mélange de dérivés acylés du saccharose contenant majoritairement le produit 6-monoacylé, la mise en réaction du dérivé 6-monoacylé du saccharose, séparé ou non des autres dérivés acylés résultant de l'étape d'acylation, avec un agent de chloration capable de chlorer les positions 1′, 4 et 6′ du 6-acylate de saccharose, la déacylation et la séparation du 4,1′,-6′-trichloro-4,1′,6′-tridésoxy-galactosaccharose formé. Selon ce procédé, la séparation du 6-acylate visé des autres acylates résultant de l'étape d'acylation peut être effectuée avant ou après l'étape de chloration, mais de la façon la plus préférable, la séparation est effectuée sur le mélange réactionnel résultant de l'acylation contenant un mélange de monoacylates ainsi que des acylates supérieures, pour donner une fraction qui est composée de, ou riche en, 6-acylate visé. Il faut sinon séparer le TGS du mélange déacylé des dérivés chlorés du saccharose obtenus.

Ce procédé présente donc l'inconvénient de nécessiter une étape de séparation, toujours longue, délicate et coûteuse, que cette étape soit effectuée avant ou après l'étape de chloration. La présente invention pallie cet inconvénient et fournit un procédé de préparation du 4,1′,6′-trichloro-4,1′6′-tridésoxy-galactosaccharose (TGS) par protection du saccharose en position 6, chloration dans les positions 4, 1′ et 6′ avec inversion de configuration au niveau du carbone 4 et déprotection de la position 6 pour donner le TGS visé, dans lequel ladite protection du saccharose en position 6 est effectuée au moyen d'une réaction d'acylation du saccharose, caractérisé par le fait que ladite réaction d'acylation est effectuée avec un système réactif composé d'une phosphine, d'un ester de type azodicarboxylique et d'un composé de type acide.

Des systèmes de ce type, connus sous le nom de réactifs de MITSUNOBU (Synthesis 1981,1) ont déjà été utilisés dans la chimie des sucres et même dans le cas du saccharose pour conduire à des thiosaccharoses (Aust. J. Chem., 1984, 37, 1925-30), à des époxysucroses (Carbohydr. Res., 1983, 121, 109-10), à des esters gras de saccharose (J. Chem. Soc. Chem. Commun. 1984, 385) ou à des aminosucres (J. Carbohydr. Chem., 1988, 7,21).

Ces publications ont fait mention de la capacité d'un tel système à réagir avec les positions primaires du saccharose, principalement les OH en position 6 et 6′. Les auteurs de la publication précitée J. Chem. Soc. Chem. Commun., 1984, 385 ont même remarqué, en préparant du 6,6′-dipalmitate de saccharose par réaction de saccharose, d'acide palmitique, de triphénylphosphine et d'azodicarboxylate de diisopropyle dans du N,N-diméthylformamide, que la position 6 était plus réactive que la position 6′ et ils ont montré qu'en traitant du saccharose par de l'acide palmitique, de la triphénylphosphine et du DIAD dans du DMF on pouvait obtenir le dérivé 6-palmitate plutôt que le dérivé 6′-palmitate. Cette observation n'a cependant pas été appliquée jusqu'à présent à la préparation du TGS qui est pourtant un produit appelé à avoir un important développement industriel.

Selon la présente invention, on fournit un procédé de préparation du 4,1′,6′-trichloro-4,1′6′-tridésoxy-galactosaccharose (TGS) à partir de saccharose par protection dudit saccharose en position 6, chloration dans les positions 4, 1′ et 6′ avec inversion de configuration au niveau du carbone 4 suivie de la déprotection de la position 6, dans lequel ladite protection du saccharose en position 6 est effectuée au moyen d'une réaction d'acylation, caractérisé par le fait que ladite réaction d'acylation est effectuée avec un système réactif composé d'une phosphine de formule générale $PR_3$, dans laquelle les groupements R, identiques ou différents, sont choisis parmi les groupements alkyle et les groupements aryle substitués ou non substitués, d'un ester azodicarboxylique de formule générale R′OCO-N=N-COOR″, dans laquelle R′ et R″, identiques ou différents, sont chacun choisis parmi les groupements alkyle et les groupements aryle, et d'un composé de type acide.

Dans un mode de réalisation préféré de l'invention, R est soit le groupement n-butyle $[(CH_2)_3- CH_3]$ soit le groupement phényle $(C_6H_5)$ ; R′ et R″ sont identiques et choisis parmi les groupements éthyle $(C_2H_5)$ et isopropyle $(i-C_3H_7)$ et le composé de type acide est du type carboxylique, aliphatique ou aromatique, un composé de type acide particulièrement préféré étant choisi parmi l'acide benzoïque et ses dérivés.

La réaction d'acétylation selon la présente invention est effectuée en milieu solvant organique, de préférence de type aprotique polaire, en particulier le N,N-diméthylformamide (DMF) ou la pyridine.

L'ordre d'addition des réactifs n'est pas déterminant, mais de préférence l'ester azodicarboxylique est ajouté au mélange des autres composants, en d'autres termes, selon ce mode de réalisation préféré de l'invention, on mélange d'abord le saccharose, la phosphine et le composé de type acide dans le solvant organique et l'on ajoute ensuite l'ester azodicarboxylique.

Les rapports stoéchiométriques de chacun des réactifs par rapport au saccharose varient entre environ 0,8 équivalent et environ 4 équivalents quand un excès d'un ou plusieurs des réactifs est souhaitable.

La température varie d'environ - 78°C à environ 80°C et les temps de réaction peuvent aller de quelques secondes (l'utilisation de réacteurs continus pouvant même être envisagée) à plusieurs heures.

L'homme de l'art comprendra aisément que plus les rapports stoéchiométriques des réactifs par rapport au saccharose sont élevés, moins les temps de réaction sont longs, autrement dit la proportion des réactifs par rapport au saccharose varie d'une façon inversement proportionnelle au temps de réaction (mais non de façon linéaire).

L'homme de l'art déterminera facilement, à l'aide de moyens analytiques bien connus tels l'analyse chromatographique, les paramètres réactionnels les uns par rapport aux autres de façon à éviter la formation d'acylates supérieurs. Ainsi, par exemple, pour des rapport stoéchiométriques donnés des différents réactifs par rapport au saccharose, le temps au bout duquel la réaction devra être arrêtée sera aisément déterminé dans chaque cas particulier.

Le produit obtenu peut être isolé par des méthodes classiques, comme par exemple extraction liquide-liquide, cristallisation, chromatographie, et les réactifs non-réagis peuvent être recyclés et les produits secondaires de la réaction régénérés. L'étape de chloration est effectuée, de façon conue en soi, à l'aide d'un agent de chloration qui peut être tout réactif capable de chlorer le 6-O-acylsaccharose dans les positions 1′, 4 et 6′, de préférence un réactif de Vilsmeier, par exemple un chlorure de N,N-dialkyl-(chlorométhaniminium) de formule générale $[XClC = N^+(R_1)_2]$ $Cl^-$ ou $R_1$ représente un groupement alkyle, en général un groupement méthyle ou éthyle et X représente un atome d'hydrogène ou un groupement méthyle. De tels réactifs sont préparés par réaction d'un chlorure d'acide avec un N,N-dialkylformamide ou un N,N-dialkylacétamide ; le chlorure d'acide pouvant par exemple être $PCl_5$, $COCl_2$ ou $SOCl_2$. L'agent de chloration peut être préparé in situ ou bien préalablement isolé. Il est aussi possible d'utiliser un autre agent de chloration tel $SO_2Cl_2$ qui forme d'abord des chlorosulfates qui peuvent être transformés en chlorodésoxysucres. La chloration du saccharose monoprotégé selon la présente invention peut être effectuée dans un solvant organique classique, en particulier le DMF, et dans une large gamme de température allant d'environ - 80°C à environ 140°C. La réactif de Vilsmeier se trouve généralement on excès par rapport au saccharose. Le mélange résultant de la réaction de chloration est ensuite neutralisé et le chlorosaccharose acylé est extrait à l'aide d'un solvant organique, précipité ou chromatographié, de façon conue en soi.

L'étape finale de déprotection de la position 6, en l'occurrence la déacylation, est effectuée de façon connue en soi par traitement basique, par exemple avec une résine basique ou avec la méthylate de sodium dans le méthanol ou avec de la soude aqueuse pour conduire au TGS visé.

L'invention est illustrée de façon plus détaillée, mais non limitative, par les Exemples suivants dans lesquels les abréviations ont le sens usuel dans l'art.

Les Exemples 1 et 2 illustrent la protection de la position 6 du saccharose selon la présente invention, les Exemples 3 et 4 illustrent respectivement les étapes, connues en elles mêmes, de chloration et de déacylation, conduisant au TGS visé.

## Exemple 1

### Protection de la position 6 du saccharose

Le saccharose (1g ; 3 mmoles), la triphénylphosphine (1,1 g ; 4,2 mmoles) et l'acide benzoïque (0,44 g ; 3,6 mmoles) sont dissous dans 10 ml de N,N-diméthylformamide (DMF) anhydre. Après ajout d'une certaine quantité de tamis moléculaire, la solution est refroidie à 0°C et l'azodicarboxylate de diisopropyle (DIAD) (0,78 ml ; 4,2 mmoles) est ajouté goutte à goutte sous agitation. Le milieu réactionnel est agité pendant 48 heures à 25°C puis concentré sous vide. Le sirop obtenu est traité par un mélange eau/dichlorométhane (1/1) et les deux phases sont séparées. La phase aqueuse contenant le saccharose non-réagi et le 6-O-benzoyl-α-D-glu-

syl-β- D -fructofuranose est évaporée et traitée par de l'éthanol anhydre. Le saccharose non-réagi précipite et peut être recyclé. Après plusieurs lavages à l'éthanol, on obtient le 6-O-benzoylsaccharose (0,584 g ; rendement = 43 %). $[\alpha]_{\underline{D}}^{20}$ = 47.3° (c = 1, MeOH).

| Anal. calc. pour $C_{19}H_{26}O_{12}.H_2O$ | C 49,14 | H 6,08 |
|---|---|---|
| trouvé | C 49,05 | H 6,22 |

RMN $^1H$ (300 MHz, $C_5D_5N$ + $D_2O$, δ p.p.m.) : 6,24 (d, 1H, $J_{1,2}$ = 3,8 Hz, H-1) 5,21 - 4,94 (m, 5H, H-6a, H-6b, H-3', H-4' et H-5) ; 4,75 (dd, 1H, H-3) ; 4.60 (m, 1H, H-5') ; 4,47 - 3,70 (m, 4H, H-1'a, H-1'b, H-6'a, H-6'b) ; 4,28-4,22 (m, 2H, H-2, H-4). RMN $^{13}C$ ($C_5D_5N$, δ p.p.m.) : 167 (C=0) ; 129 - 134 (Ar) ; 105,7 (C-2') ; 93,2 ( C-1 ) ; 84,2 (C-5') ; 79,6 - 75,6 - 74,8 - 73,2      -71,8 - 71,4      (C-3' - C-4' - C-3 - C-5 - C-2 - C-4) ; 64,9 - 64,6 -63,3 (C-6 - C-6' - C-1').

La structure est ensuite confirmée par hydrolyse enzymatique à l'aide d'une invertase conduisant à du fructose et du 6-O-benzoylglucose.

## Exemple 2

**Protection de la position 6 du saccharose**

Le saccharose (5 g ; 15 mmoles), la triphénylphosphine (16,3 g ; 62 mmoles) et l'acide benzoïque (7,4 g ; 60 mmoles) sont dissous dans 120 ml de N,N-diméthylformamide (DMF) anhydre additionnés de tamis moléculaire. Le DIAD (11,5 ml ; 62 mmoles) est ajouté en une seule fois sans refroidissement préalable de la solution. Après 2 mn de réaction, 10 ml d'eau sont ajoutées pour arrêter la réaction et le solvant est évaporé sous pression réduite. Le sirop obtenu est traité par de l'éthanol dans lequel précipitent certains produits secondaires qui sont ensuite éliminés par filtration. La solution éthanolique est évaporée et traitée par un mélange eau/dichlorométhane (1/1). La phase aqueuse contenant le 6-O-benzoylsaccharose est évaporée et chromatographiée sur silice avec un éluant composé de chloroforme : acétone : méthanol : eau, 56 : 20 : 20 : 4. Le 6-O-benzoylsaccharose est obtenu avec un rendement de 80 %.

## Exemple 3

**Chloration**

Cette étape de chloration a été effectuée de façon connue en soi comme suit.

Le 6-O-benzoylsaccharose (1g ; 2,2 mmoles) est dissous dans 10 ml de DMF. Le chlorure de sulfuryle (5,3 ml ; 6,6 mmoles) est ajouté à - 40°C et le mélange est agité pendant 4 heures. On laisse la température revenir aux conditions ambiantes et on poursuit l'agitation jusqu'à obtention d'un maximum de produit trichloré (suivi en CCM).

Le mélange est coulé dans de l'eau glacée acidifiée à l'acide sulfurique et une extraction répétée au chloroforme permet d'obtenir, après lavage au bicarbonate et à l'eau neutre, une solution qui, après évaporation, donne un sirop. Ce sirop est dissous dans du méthanol et la solution est déchlorosulfatée à l'aide d'une quantité catalytique de NaI. Après filtration et neutralisation, la solution est évaporée et chromatographiée sur silice pour donner du 6-O-benzoyl - 4,1',6'-trichloro-4,1',6'-tridésoxy-galactosaccharose (0,57 g ; rendement = 52 %).

## Exemple 4

**Débenzoylation**

Cette étape de déacylation a été effectuée de façon connue en soi comme suit.

Le 6-O-benzoyl-4,1',6'-trichloro-4,1',6'-tridésoxy-galactosaccharose obtenu dans l'Exemple 3 est dissous dans du méthanol et débenzoylé avec du méthylate de sodium (pH = 10) pendant 12 heures. Après neutralisation à l'aide d'une résine acide, filtration et évaporation, on obtient le 4,1',6' -trichloro-4,1',6'-tridésoxygalactosaccharose (TGS) (0,434 g ; rendement = 96 %) dont les caractéristiques physiques sont identiques à celles décrites dans la littérature.

## Revendications

1. Procédé de préparation du 4,1',6'-trichloro-4,1',6'-tridésoxy-galactosaccharose (TGS) à partir de saccharose par protection dudit saccharose en position 6, chloration dans les positions 4, 1' et 6' avec inversion de configuration au niveau du carbone 4 suivie de la déprotection de la position 6, dans lequel ladite protection du saccharose en position 6 est effectuée au moyen d'une réaction d'acylation, caractérisé par le fait que ladite réaction d'acylation est effectuée avec un système réactif composé d'une phosphine de formule générale $PR_3$, dans laquelle les groupements R, identiques ou différents, sont choisis parmi les groupements alkyle et les groupements aryle substitués ou non substitués, d'un ester azodicarboxylique de formule générale R'OCO-N=N-COOR", dans laquelle R' et R", identiques ou différents, sont chacun choisis parmi les groupements alkyle et les groupements aryle et d'un composé de type acide, en milieu solvant organique.

2. Procédé selon la revendication 1, caractérisé par le fait que lesdits radicaux R de ladite phosphine de formule générale $PR_3$ sont identiques et choisis parmi les groupements n-butyle et phényle.

3. Procédé selon l'une des revendications 1 et 2, caractérisé par le fait que lesdits radicaux R' et R" dudit ester azodicarboxylique de formule générale R'OCO-N=N-COOR" sont identiques et choisis parmi les groupements éthyle et isopropyle.

4. Procédé selon l'une quelconque des revendications 1 à 3, caractérisé par le fait que ledit composé de type acide est du type carboxylique, aliphatique ou aromatique.

5. Procédé selon la revendication 4, caractérisé par le fait que ledit composé du type acide est choisi parmi l'acide benzoïque et ses dérivés.

6. Procédé selon l'une quelconque des revendications 1 à 5, caractérisé par le fait que ledit solvant organique est un solvant de type aprotique polaire.

7. Procédé selon la revendication 6, caractérisé par le fait que ledit solvant organique est choisi parmi le N-N-diméthylformamide et la pyridine.

8. Procédé selon l'une quelconque des revendications 1 à 7, caractérisé par le fait que chacun des rapports stoéchiométriques de ladite phosphine, dudit ester azodicarboxylique et dudit composé de type acide au saccharose est compris entre environ 0,8 et environ 4.

9. Procédé selon l'une quelconque des revendications 1 à 8, caractérisé par le fait que ledit ester azodicarboxylique est ajouté à un mélange contenant ledit saccharose, ladite phosphine et ledit composé de type acide.